(19) Europäisches Patentamt
European Patent Office
Office européen des brevets

(11) EP 1 923 408 A1

(12) DEMANDE DE BREVET EUROPEEN

(43) Date de publication:
**21.05.2008 Bulletin 2008/21**

(51) Int Cl.:
***C08F 265/04*** *(2006.01)* ***C08F 293/00*** *(2006.01)*
***A61K 8/72*** *(2006.01)* ***A61K 8/90*** *(2006.01)*

(21) Numéro de dépôt: **07118609.2**

(22) Date de dépôt: **16.10.2007**

(84) Etats contractants désignés:
**AT BE BG CH CY CZ DE DK EE ES FI FR GB GR HU IE IS IT LI LT LU LV MC MT NL PL PT RO SE SI SK TR**
Etats d'extension désignés:
**AL BA HR MK RS**

(30) Priorité: **16.11.2006 FR 0654944**

(71) Demandeur: **L'Oréal**
**75008 Paris (FR)**

(72) Inventeur: **Farcet, Céline**
**75012, PARIS (FR)**

(74) Mandataire: **Dodin, Catherine**
**L'OREAL**
**DIPI**
**25-29 Quai Aulagnier**
**92665 Asnières-sur-Seine Cedex (FR)**

# (54) Nouveaux polymères séquencés, compositions les comprenant et procédé de traitement cosmétique

(57) L'invention concerne de nouveaux polymères séquencés comprenant des monomères siliconés particuliers et des monomères de formule $CH_2=C(R1)-COOR2$ dans laquelle R1 est H ou $CH_3$ et R2 représente un groupe cycloalkyle en C4 à C12.

L'invention concerne également les compositions cosmétiques ou pharmaceutiques comprenant ces polymères, ainsi qu'un procédé de traitement cosmétique les employant.

EP 1 923 408 A1

## Description

La présente invention a trait à de nouveaux polymères de structure spécifique ainsi qu'aux compositions cosmétiques comprenant de tels polymères. L'invention concerne également un procédé de traitement cosmétique employant lesdits polymères.

Divers types de polymères sont conventionnellement utilisés dans les compositions cosmétiques en raison des diverses propriétés qu'ils peuvent leur apporter. Ils sont par exemple utilisés dans des compositions de maquillage ou de soin de la peau, des lèvres ou des phanères, telles que des vernis à ongles ou des compositions pour les cheveux.
Cependant, en utilisant au sein d'une même composition deux polymères incompatibles, c'est à dire non miscibles dans un même solvant, le formulateur est confronté, du fait de l'incompatibilité des polymères, à des problèmes de séparation de phases, voire de décantation, et de manière générale à l'obtention d'une composition non homogène. Ces problèmes ne pouvaient être jusqu'ici résolus que par la présence dans la composition d'un composé permettant de compatibiliser les polymères entre eux.
Pour pallier ce problème, il a été proposé, dans la demande EP1411069, des polymères de structure particulière, comprenant une première et au moins une deuxième séquence, incompatibles entre elles, et reliées par une séquence intermédiaire qui comprend au moins un monomère constitutif de chacune desdites deux séquences.
Cette demande décrit principalement de polymères séquencés préparés à partir de monomères de type acrylates et méthacrylates d'alkyle, notamment de méthyle, d'isobutyle, d'isobornyle, de trifluoroéthyle, ou d'acide (méth)acrylique. Il est également mentionné, d'une manière générale, la possibilité d'incorporer des monomères additionnels siliconés dans le polymère. Toutefois, aucun avantage particulier n'est mentionné en liaison avec cette possibilité.

Les polymères décrits dans l'art antérieur présentent toutefois comme inconvénient d'être sensibles aux agressions par des corps gras extérieurs, tels que par exemple par les huiles alimentaires ou le sébum.
Il en résulte que le dépôt est altéré lors de ces agressions, et devient collant. La composition présente donc une moins bonne tenue.
Par ailleurs, on a constaté que le confort de la composition est également diminué; en effet, plus un dépôt, ou film, est sensible aux huiles, donc collant, plus il sera inconfortable à porter.

La présente invention a pour but de proposer de nouveaux polymères pouvant permettre l'obtention de compositions confortables à porter, de bonne tenue et peu collantes, résistants aux agressions par les corps gras tels que les huiles, tout en étant véhiculables dans les solvants carbonés bien qu'elles comprennent des monomères siliconés, et en étant brillants.

Ainsi, un objet de la présente invention est un polymère séquencé comprenant au moins une première séquence et au moins une deuxième séquence, dans lequel :

- au moins l'une des séquences, dite première séquence, comprend de 0,5 à 35% en poids, par rapport au poids de ladite première séquence, d'au moins un monomère siliconé choisi parmi, seul ou en mélange, les monomères tels que décrits ci-après;
- au moins une séquence, identique ou différente de ladite première séquence, comprend de 0,5 à 100% en poids, par rapport au poids de ladite séquence, d'au moins un monomère choisi parmi, seul ou en mélange, les monomères de formule (I) : $CH_2=C(R1)-COOR2$ dans laquelle R1 est H ou $CH_3$ et R2 représente un groupe cycloalkyle en C3 à C12, notamment en C6-C10.

L'invention a également pour objet une composition notamment cosmétique ou pharmaceutique comprenant, dans un milieu physiologiquement acceptable, un tel polymère.

On a en effet constaté que les polymères selon l'invention étaient de manière surprenante plus confortables que ceux de l'art antérieur ne comprenant pas de monomères siliconés.
Ces films sont également moins cassants que ceux de l'art antérieur.
On a également constaté que les polymères selon l'invention, bien qu'ils comprennent des monomères siliconés, présentent une bonne solubilité dans les corps gras carbonés, que cela soit dans les huiles cosmétiques ou les solvants de type esters courts, solubilité qui peut varier et être ajustée selon la nature et/ou la quantité des monomères utilisés.
Cette bonne liposolubilité peut faciliter leur mise en oeuvre ultérieure, notamment dans les compositions cosmétiques qui comprennent généralement une phase grasse.
Par ailleurs, les polymères selon l'invention permettent l'obtention de films plus ou moins brillants, selon la nature et la proportion des monomères employés.
Enfin, la présence de monomères siliconés, même en faible proportion, peut permettre d'obtenir des polymères présentant une viscosité plus basse que celle des polymères de l'art antérieur, ce qui facilite leur mise en oeuvre dans les compositions cosmétiques.

Les polymères selon la présente invention sont des polymères séquencés, comprenant au moins une première séquence et au moins une deuxième séquence, ayant des températures de transition vitreuse (Tg) différentes, lesdites

première et deuxième séquences étant avantageusement reliées entre elles par un segment intermédiaire comprenant au moins un monomère constitutif de la première séquence et au moins un monomère constitutif de la deuxième séquence.

On précise que dans ce qui précède et ce qui suit les termes "première" et "deuxième" séquences ne conditionnent nullement l'ordre desdites séquences (ou blocs) dans la structure du polymère.

Le polymère séquencé selon l'invention comprend donc au moins une première séquence et au moins une deuxième séquence, ces séquences étant avantageusement incompatibles l'une avec l'autre.

Par "séquences incompatibles l'une avec l'autre", on entend que le mélange formé du polymère correspondant à la première séquence et du polymère correspondant à la deuxième séquence, n'est pas miscible dans le solvant de polymérisation majoritaire en poids du polymère séquencé, à température ambiante (25°C) et pression atmosphérique ($10^5$ Pa), pour une teneur du mélange de polymères supérieure ou égale à 5% en poids, par rapport au poids total du mélange (polymères et solvant), étant entendu que :

i) lesdits polymères sont présents dans le mélange en une teneur telle que le rapport pondéral respectif va de 10/90 à 90/10, et que

ii) chacun des polymères correspondant à la première et seconde séquences a une masse moléculaire moyenne (en poids ou en nombre) égale à celle du polymère séquencé +/- 15%.

Dans le cas d'un mélange de solvants de polymérisation, dans l'hypothèse de deux ou plusieurs solvants présents, ledit mélange de polymères est non miscible dans au moins l'un d'entre eux. Bien entendu, dans le cas d'une polymérisation réalisée dans un solvant unique, ce dernier est le solvant majoritaire.

Lesdites première et deuxième séquences peuvent être avantageusement reliées entre elles par un segment intermédiaire comprenant au moins un monomère m1 constitutif de la première séquence et au moins un monomère m2 constitutif de la deuxième séquence.

Le segment intermédiaire forme une séquence (ou bloc). De préférence, m2 est différent de m1. Le segment ou séquence intermédiaire peut permettre de "compatibiliser" ces première et deuxième séquences.

Le polymère séquencé de la composition selon l'invention est avantageusement un polymère éthylénique séquencé, linéaire, ramifié ou greffé, de préférence formant un dépôt, et plus particulièrement filmogène.

Par polymère "éthylénique" , on entend un polymère obtenu par polymérisation de monomères comprenant une insaturation éthylénique.

Par polymère "séquencé", on entend un polymère comprenant au moins 2 séquences distinctes, de préférence au moins 3 séquences distinctes; de préférence diblocs ou triblocs.

Par polymère "formant un dépôt", on entend un polymère apte à former à lui seul ou en présence d'un agent auxiliaire, un dépôt adhérent sur un support, notamment sur les matières kératiniques.

Par polymère "filmogène", on entend un polymère apte à former à lui seul ou en présence d'un agent auxiliaire de filmification, un film continu et adhérent sur un support, notamment sur les matières kératiniques.

Chaque séquence, ou bloc, du polymère selon l'invention est issue d'un type de monomère ou de plusieurs types de monomères différents. Cela signifie que chaque séquence peut être constituée d'un homopolymère ou d'un copolymère, qui peut être statistique, alterné ou autre.

Avantageusement, lorsqu'il est présent, le segment intermédiaire comprenant au moins un monomère constitutif de la première séquence et au moins un monomère constitutif de la deuxième séquence du polymère est un polymère statistique. De préférence, la séquence intermédiaire est issue essentiellement de monomères constitutifs de la première séquence et de la deuxième séquence. Par "essentiellement", on entend au moins à 85%, de préférence au moins à 90%, mieux à 95% et encore mieux à 100%.

Selon l'invention, les première et deuxième séquences ont de préférence des températures de transition vitreuse différentes, avec un écart entre les températures de transition vitreuse des première et deuxième séquences généralement supérieur à 5°C, de préférence supérieur à 10°C, et mieux supérieur à 20°C.

Avantageusement, la séquence intermédiaire a une température de transition vitreuse Tg comprise entre les températures de transition vitreuse des première et deuxième séquences.

Les températures de transition vitreuse indiquées sont, sauf indication contraire, des Tg théoriques déterminées à partir des Tg théoriques des monomères constitutifs de chacune des séquences, que l'on peut trouver dans un manuel de référence tel que le Polymer Handbook, 4th ed. (Brandrup, Immergut, Grulke), 1999, John Wiley, selon la relation suivante, dite Loi de Fox :

$$\frac{1}{Tg} = \sum_i \left(\frac{\varpi i}{Tgi}\right)$$

wi étant la fraction massique du monomère i dans la séquence considérée et Tgi étant la température de transition vitreuse de l'homopolymère du monomère i (exprimée en Kelvin).

Le polymère selon l'invention se caractérise par le fait qu'au moins l'une de ses séquences, dite première séquence, comprend de 0,5 à 35% en poids, par rapport au poids de ladite première séquence, d'au moins un monomère siliconé choisi parmi, seul ou en mélange, les monomères suivants :

- (i) les monomères éthyléniques dont le groupement ester contient des silanes et/ou des siloxanes, de formule :

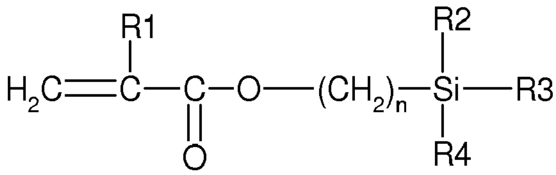

dans laquelle :

- R1= H ou méthyle,
- R2, R3, R4, identiques ou différents, représentent des groupes alkyles en C1-C6 ou le groupe $-OSi(R5)_3$ avec R5= méthyle ou éthyle; de préférence R2, R3 et/ou R4 représentent, indépendamment l'un de l'autre, $-OSi(Me)_3$ et/ou méthyle;
- n est un nombre entier de 1 à 10, de préférence égal à 1 ou 3.
- (ii) les macromonomères PDMS, ou polydiméthylsiloxanes à groupement terminal monoacryloyloxy ou monométhacryloyloxy, de formule suivante

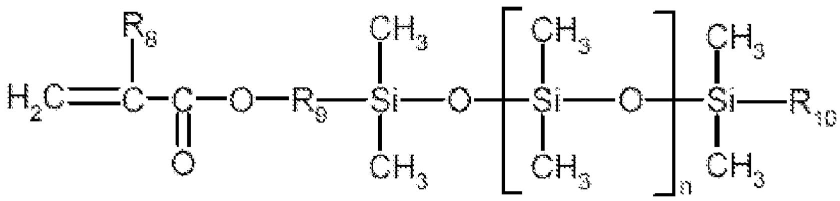

dans laquelle :

- R8 désigne un atome d'hydrogène ou un groupement méthyle; de préférence méthyle;
- R9 désigne un groupe hydrocarboné divalent, linéaire ou ramifié, de préférence linéaire, ayant de 1 à 10 atomes de carbone et contenant éventuellement une ou deux liaisons éther -O- ; de préférence éthylène, propylène ou butylène;
- R10 désigne un groupe alkyle linéaire ou ramifié, ayant de 1 à 10 atomes de carbone, notamment de 2 à 8 atomes de carbone; de préférence méthyle, éthyle, propyle, butyle ou pentyle;
- n désigne un nombre entier allant de 1 à 300, de préférence allant de 3 à 200, et préférentiellement allant de 5 à 100.
- (iii) les monomères éthyléniques dont le groupement ester contient des carboxysilanes dendrimères de formule :

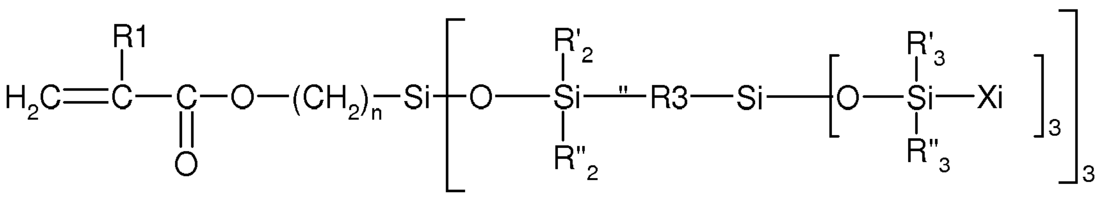

dans laquelle :

- R1= H ou méthyle;

- n est un nombre entier de 1 à 10, de préférence égal à 1, 2, 3 ou 4;
- R’2, R"2, R’3 et R"3, identiques ou différents, représentent un groupe alkyle en C1 à C10, de préférence méthyle ou éthyle;
- R3 est un groupe divalent alkylène en C2 à C10, de préférence en C2 ou C3;
- i est un entier compris entre 1 et 10, notamment i = 1, 2 ou 3;
- quand i = 2 à 10, X(i), identique ou différent, représente un groupe -R4-Si-[O-(R’3)(R"3)-X(i-1)]$_3$, avec R4, identique ou différent, représentant un groupe divalent alkylène en C2 à C10, de préférence en C2 ou C3;
- et X(1) représente H ou un groupe alkyle en C1-C10, notamment méthyle ou éthyle; et en particulier les monomères suivants, dans lesquels R1 = H ou méthyle :

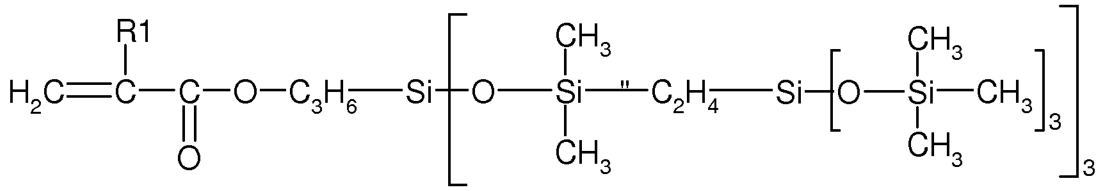

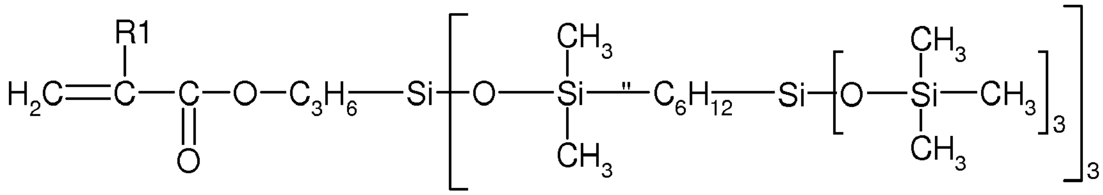

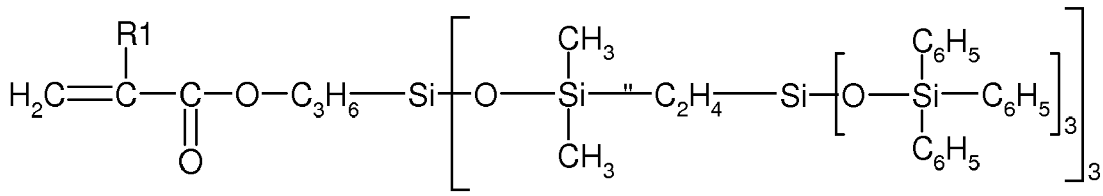

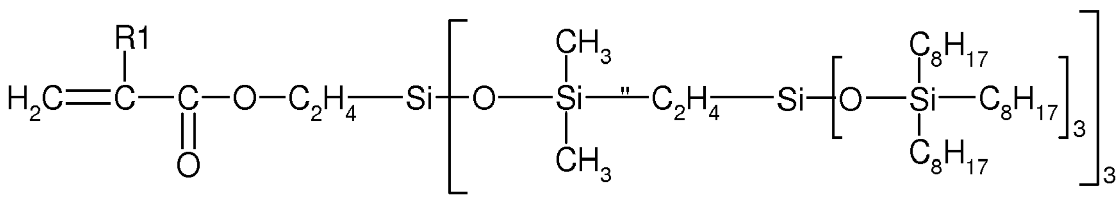

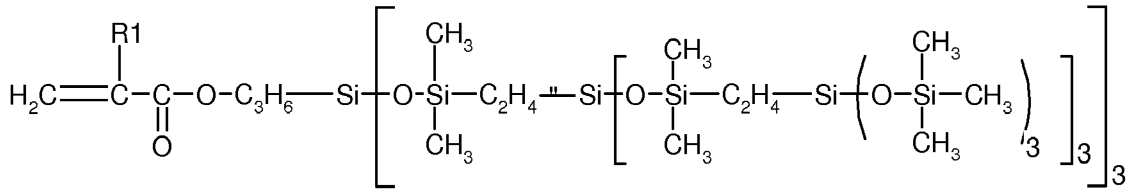

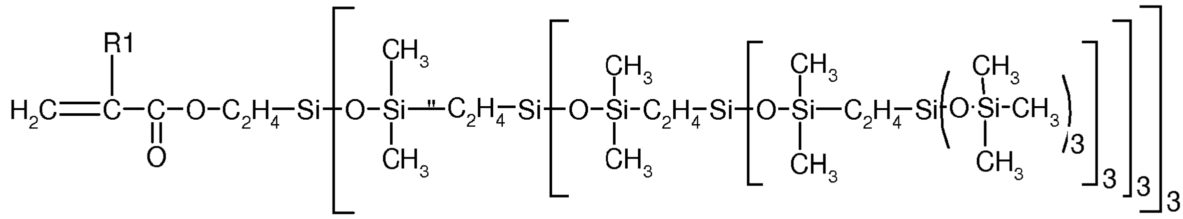

- (iv) les monomères éthyléniques de type POSS ou POS de structure :

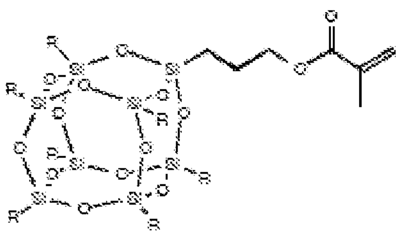
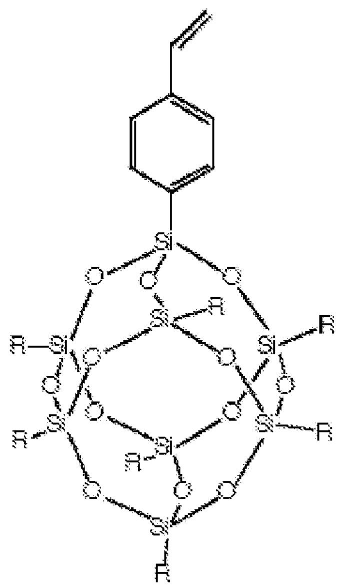

dans lesquelles R, identique ou différent, est un groupe alkyle linéaire en C1 à C10, de préférence méthyle, ou cyclique en C3 à C12, de préférence en C5.

On peut en particulier citer les monomères suivants: le (méth)acryloxypropyltris(trimethylsiloxy)silane, le (méth) acryloxypropylbis(trimethylsiloxy)méthylsilane, le (méth)acryloxyméthyltris(trimethylsiloxy)-silane, le (méth)acryloxyméthylbis(trimethylsiloxy)méthylsilane; le (méth)acryloxypropryltrimethoxysilane; et tout particulièrement les monomères suivants :

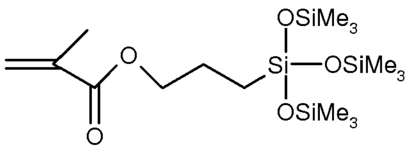

méthacryloxypropyltris(trimethylsiloxy)silane

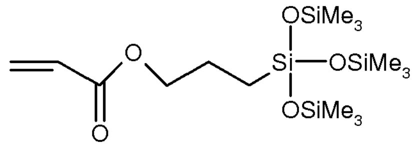

acryloxypropyltris(trimethylsiloxy)silane

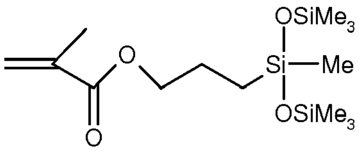

méthacryloxypropylbis(trimethylsiloxy)méthylsilane

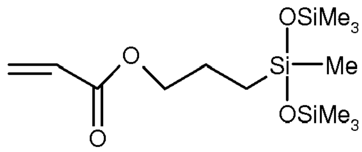

acryloxypropylbis(trimethylsiloxy)méthylsilane

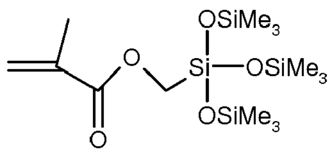

methacryloxymethyltris(trimethylsiloxy)silane

On peut également citer les monomères (méth)acrylique de type POSS (polyhedral oligomeric silsesquioxanes) et POS (polyhedral polymeric silicates), notamment d'Hybrid Plastics; et les monométhacryloyloxypropyl polydiméthylsiloxanes tels que ceux commercialisés sous la dénomination PS560-K6 par UCT (United Chemical Technologies Inc.) ou sous la dénomination MCR-M17 par Gelest Inc.

Les monomères siliconés ci-dessus représentent 0,5% à 35% en poids, notamment 1 à 30% en poids, voire 1,5 à 25% en poids, encore mieux 2 à 20% en poids, et préférentiellement 3 à 10% en poids, du poids total de la séquence les comprenant.

Ladite séquence comprend donc en outre 65% à 99,5% en poids, notamment 70 à 99% en poids, voire 75 à 98,5% en poids, encore mieux 80 à 98% en poids, préférentiellement 90 à 97% en poids, par rapport au poids total de ladite séquence, d'un ou plusieurs autres monomères, notamment choisis parmi les monomères additionnels tels que définis ci-après, et/ou les monomères de formule (I).

Le polymère selon l'invention se caractérise également par le fait qu'au moins une séquence, identique ou différente de la première séquence ci-dessus, comprend de 0,5 à 100% en poids, par rapport au poids de ladite séquence, d'au moins un monomère choisi parmi, seul ou en mélange, les monomères de formule (I) : $CH_2$=C(R1)-COOR2 dans laquelle R1 est H ou $CH_3$ et R2 représente un groupe cycloalkyle en C4 à C12, notamment en C6-C10.

Ainsi, les monomères siliconés et les monomères de formule (I) peuvent être présents dans la même séquence ou dans des séquences différentes. De préférence, ils sont présents dans des séquences différentes.

Lorsqu'ils sont présents dans la même séquence que les monomères siliconés, le ou les monomères de formule (I) représentent de préférence 0,5 à 99,5% en poids, notamment 5 à 99% en poids, voire 20 à 90% en poids, encore mieux 40 à 80% en poids, préférentiellement 50 à 70% en poids, du poids total de la séquence les comprenant.

Lorsqu'ils sont présents dans une séquence différente de celle comprenant les monomères siliconés, le ou les monomères de formule (I) représentent de préférence 0,5 à 100% en poids, notamment 5 à 99% en poids, voire 20 à 90% en poids, encore mieux 40 à 80% en poids, préférentiellement 50 à 70% en poids, du poids total de la séquence les comprenant.

De préférence, les monomères de formule (I) sont choisis parmi le méthacrylate d'isobornyle, l'acrylate d'isobornyle, l'acrylate et le méthacrylate de cyclohexyle, l'acrylate et le méthacrylate de t-butylcyclohexyle, et leurs mélanges.

De préférence, la séquence comprenant les monomères de formule (I) comprend à la fois du méthacrylate d'isobornyle et de l'acrylate d'isobornyle.

De préférence également, ladite séquence ne comprend pas de monomères additionnels, et préférentiellement ne comprend que du méthacrylate et de l'acrylate d'isobornyle.

De préférence, le méthacrylate d'isobornyle est présent à raison de 25% à 75% en poids, notamment 30 à 70% en poids, voire 40 à 60% en poids, par rapport au poids de la séquence le comprenant; de préférence, l'acrylate d'isobornyle est présent à raison de 25% à 75% en poids, notamment 30 à 70% en poids, voire 40 à 60% en poids, par rapport au poids de la séquence le comprenant.

Lorsque les monomères de formule (I) et/ou les monomères siliconés ne sont pas présents à 100% en poids dans la/les séquences les comprenant, celle(s)-ci peu(ven)t donc comprendre un ou plusieurs monomères additionnels tels que définis ci-après.

Le polymère selon l'invention peut également comprendre une ou plusieurs séquences ne comprenant que des monomères additionnels; ceci peut notamment être le cas lorsque les monomères de formule (I) et les monomères siliconés sont présents dans la même séquence.

Les monomères additionnels susceptibles d'être présents dans les séquences peuvent être identiques ou différents selon les séquences ou au sein d'une même séquence.

Ils peuvent en particulier être choisis parmi, seul ou en mélange, les monomères suivants :

- (i) les hydrocarbures éthyléniques ayant 2 à 10 carbones, tels que l'éthylène, l'isoprène, ou le butadiène ;

- (ii) les (méth)acrylates de formule $CH_2 = CHCOOR'_3$ ou $CH_2 = C(CH_3)COOR'_3$

dans lesquelles $R'_3$ représente :

- un groupe alkyle linéaire ou ramifié, de 1 à 22 atomes de carbone, notamment 4 à 20, voire 6 à 18 atomes de carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi les groupes hydroxyle ou les atomes d'halogène (Cl, Br, I et F);
  notamment R'3 peut être un groupe méthyle, éthyle, propyle, n-butyle, isobutyle, tertiobutyle, hexyle, éthylhexyle, octyle, lauryle, isooctyle, isodécyle, dodécyle, béhényle, stéaryle; éthyl-2-perfluorohexyle; ou un groupe hydroxyalkyle en $C_{1-4}$ tel que 2-hydroxyéthyle, 2-hydroxybutyle et 2-hydroxypropyle; ou un groupe alcoxy $(C_{1-4})$ alkyle $(C_{1-4})$ tel que méthoxyéthyle, éthoxyéthyle et méthoxypropyle,
- un groupe aryle en $C_3$ à $C_{20}$ tel que le groupe phényle,
- un groupe aralkyle en $C_4$ à $C_{30}$ (groupe alkyle en $C_1$ à $C_8$) tel que 2-phényl-éthyle, t-butylbenzyle ou benzyle,
- un groupe hétérocyclique de 4 à 12 chaînons contenant un ou plusieurs hétéroatomes choisis parmi O, N, et S, le cycle étant aromatique ou non,
- un groupe hétérocycloalkyle (alkyle en C1-C4), tel que furfurylméthyle ou tétrahydrofurfurylméthyle,
  lesdits groupes aryle, aralkyle, hétérocyclique ou hétérocycloalkyle pouvant être éventuellement substitué par un ou plusieurs substituants choisis parmi les groupes hydroxyles, les atomes d'halogène, et les groupes alkyles en C1-4, linéaires ou ramifiés dans lesquels se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi les groupes hydroxyle ou les atomes d'halogène (Cl, Br, I et F),
- $R'_3$ peut également être un groupe $-(C_2H_4O)_m-R''$, avec m = 5 à 150 et R" = H ou alkyle de $C_1$ à $C_{30}$, par exemple -POE-méthyle ou -POE-béhényle;
- (iii) les (méth)acrylamides de formule :

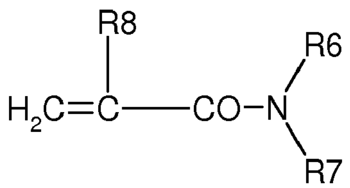

dans laquelle $R_8$ désigne H ou méthyle; et $R_7$ et $R_6$ identiques ou différents représentent :

- un atome d'hydrogène; ou
- un groupe alkyle linéaire ou ramifié, de 1 à 22 atomes de carbone, notamment 4 à 20, voire 6 à 18 atomes de

carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi les groupes hydroxyle, les atomes d'halogène (Cl, Br, I et F);
notamment R6 et/ou R7 peut être un groupe méthyle, éthyle, propyle, n-butyle, isobutyle, tertiobutyle, hexyle, éthylhexyle, octyle, lauryle, isooctyle, isodécyle, dodécyle, béhényle, stéaryle; éthyl-2-perfluorohexyle; ou un groupe hydroxyalkyle en $C_{1-4}$ tel que 2-hydroxyéthyle, 2-hydroxybutyle et 2-hydroxypropyle; ou un groupe alcoxy ($C_{1-4}$) alkyle ($C_{1-4}$) tel que méthoxyéthyle, éthoxyéthyle et méthoxypropyle,

- un groupe cycloalkyle en $C_3$ à $C_{12}$, tel que le groupe isobornyle, cyclohexyle, t-butylcyclohexyle;
- un groupe aryle en $C_3$ à $C_{20}$ tel que le groupe phényle,
- un groupe aralkyle en $C_4$ à $C_{30}$ (groupe alkyle en $C_1$ à $C_8$) tel que 2-phényl-éthyle, t-butylbenzyle ou benzyle,
- un groupe hétérocyclique de 4 à 12 chaînons contenant un ou plusieurs hétéroatomes choisis parmi O, N, et S, le cycle étant aromatique ou non,
- un groupe hétérocycloalkyle (alkyle en C1-C4), tel que furfurylméthyle ou tétrahydrofurfurylméthyle,
lesdits groupes cycloalkyle, aryle, aralkyle, hétérocyclique ou hétérocycloalkyle pouvant être éventuellement substitué par un ou plusieurs substituants choisis parmi les groupes hydroxyles, les atomes d'halogène, et les groupes alkyles en C1-C4, linéaires ou ramifiés dans lesquels se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi les groupes hydroxyle et les atomes d'halogène (Cl, Br, I et F).
Des exemples de monomères (méth)acrylamide sont le (méth)acrylamide, le N-éthyl(méth)acrylamide, le N-butylacrylamide, le N-t-butylacrylamide, le N-isopropylacrylamide, le N,N-diméthyl(méth)acrylamide, le N,N-dibutylacrylamide, le N-octylacrylamide, le N-dodécylacrylamide, l'undécylacrylamide, et le N(2-hydroxypropylméthacrylamide).
- (iv) les composés vinyliques de formules :
$CH_2=CH-R_9$, $CH_2=CH-CH_2-R_9$ ou $CH_2=C(CH_3)-CH_2-R_9$

dans lesquelles $R_9$ est un groupe hydroxyle, halogène (Cl ou F), $NH_2$, $OR_{14}$ où $R_{14}$ représente un groupe phényle ou un groupe alkyle en $C_1$ à $C_{12}$ (le monomère est un éther de vinyle ou d'allyle); acétamide ($NHCOCH_3$); un groupe $OCOR_{15}$ où $R_{15}$ représente un groupe alkyle de 2 à 12 carbones, linéaire ou ramifié (le monomère est un ester vinylique ou d'allyle); ou un groupe choisi parmi:

- un groupe alkyle linéaire ou ramifié, de 1 à 22 atomes de carbone, notamment 4 à 20, voire 6 à 18 atomes de carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi les groupes hydroxyle, les atomes d'halogène (Cl, Br, I et F),
- un groupe cycloalkyle en $C_3$ à $C_{12}$ tel que isobornyle, cyclohexyle,
- un groupe aryle en $C_3$ à $C_{20}$ tel que phényle,
- un groupe aralkyle en $C_4$ à $C_{30}$ (groupe alkyle en $C_1$ à $C_8$) tel que 2-phényléthyle ; benzyle,
- un groupe hétérocyclique de 4 à 12 chaînons contenant un ou plusieurs hétéroatomes choisis parmi O, N, et S, le cycle étant aromatique ou non,
- un groupe hétérocycloalkyle (alkyle en C1-C4), tel que furfurylméthyle ou tétrahydrofurfurylméthyle,
lesdits groupes cycloalkyle, aryle, aralkyle, hétérocyclique ou hétérocycloalkyle pouvant être éventuellement substitué par un ou plusieurs substituants choisis parmi les groupes hydroxyles, les atomes d'halogène, et les groupes alkyles de 1 à 4 C linéaires ou ramifiés dans lesquels se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi les groupes hydroxyle les atomes d'halogène (Cl, Br, I et F);
Des exemples de monomères vinyliques sont le vinylcyclohexane et le styrène.
Des exemples d'esters vinyliques sont l'acétate de vinyle, le propionate de vinyle, le butyrate de vinyle, l'éthylhexanoate de vinyle, le néononanoate de vinyle et le néododécanoate de vinyle.
Parmi les éthers de vinyle, on peut citer le vinyl méthyl éther, le vinyl éthyl éther et le vinyl isobutyl éther.
- (v) les monomères à insaturation(s) éthylénique(s) comprenant au moins une fonction acide carboxylique, phosphorique ou sulfonique, ou anhydride, comme par exemple l'acide acrylique, l'acide méthacrylique, l'acide crotonique, l'anhydride maléique, l'acide itaconique, l'acide fumarique, l'acide maléique, l'acide acrylamidopropanesulfonique, l'acide vinylbenzoïque, l'acide vinylphosphorique et les sels de ceux-ci,
- (vi) les monomères à insaturation(s) éthylénique(s) comprenant au moins une fonction amine tertiaire comme la 2-vinylpyridine, la 4-vinylpyridine, le méthacrylate de diméthylaminoéthyle, le méthacrylate de diéthylaminoéthyle, le diméthylaminopropyl méthacrylamide et les sels de ceux-ci.

Les sels peuvent être formés par neutralisation des groupes anioniques à l'aide d'une base minérale, telle que

LiOH, NaOH, KOH, $Ca(OH)_2$, $NH_4OH$ ou $Zn(OH)_2$; ou par une base organique telle qu'une alkylamine primaire, secondaire ou tertiaire, notamment la triéthylamine ou la butylamine. Cette alkylamine primaire, secondaire ou tertiaire peut comporter un ou plusieurs atomes d'azote et/ou d'oxygène et peut donc comporter par exemple une ou plusieurs fonctions alcool; on peut notamment citer l'amino-2-méthyl-2-propanol, la triéthanolamine et la diméthylamino-2-propanol. On peut encore citer la lysine ou la 3-(diméthylamino)-propylamine.

On peut également peut citer les sels d'acides minéraux, tels que l'acide sulfurique, l'acide chlorhydrique, l'acide bromhydrique, l'acide iodhydrique, l'acide phosphorique, l'acide borique. On peut aussi citer les sels d'acides organiques, qui peuvent comporter un ou plusieurs groupes acide carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique et l'acide tartrique.

Lorsque les monomères de formule (I) et les monomères siliconés sont présents dans la même séquence, on choisit plus particulièrement les éventuels monomères additionnels parmi, seuls ou en mélange, :

- les (méth)acrylates de formule $CH_2 = CHCOOR'_3$ ou $CH_2 = C(CH_3)COOR'_3$

dans lesquelles $R'_3$ représente un groupe alkyle linéaire ou ramifié, de 1 à 22 atomes de carbone, dans lequel se trouve (nt) éventuellement intercalé(s) un ou plusieurs hétéroatomes;

- les monomères à insaturation(s) éthylénique(s) comprenant au moins une fonction acide carboxylique, ou anhydride. En particulier, on peut citer l'acide acrylique, l'acide méthacrylique, et les (méth)acrylates d'isobutyle, de 2-éthylehexyle, de dodécyle, de stéaryle, de béhényle, et leurs mélanges.

Lorsque les monomères de formule (I) et les monomères siliconés ne sont pas présents dans la même séquence, on choisit plus particulièrement les éventuels monomères additionnels présents dans la séquence comprenant les monomères de formule (I) et/ou les éventuels monomères additionnels présents dans la séquence comprenant les monomères siliconés parmi, seuls ou en mélange,:

- les (méth)acrylates de formule $CH_2 = CHCOOR'_3$ ou $CH_2 = C(CH_3)COOR'_3$

dans lesquelles $R'_3$ représente un groupe alkyle linéaire ou ramifié, de 1 à 22 atomes de carbone, dans lequel se trouve (nt) éventuellement intercalé(s) un ou plusieurs hétéroatomes;

- les monomères à insaturation(s) éthylénique(s) comprenant au moins une fonction acide carboxylique, ou anhydride. En particulier, on peut citer l'acide acrylique, l'acide méthacrylique, et les (méth)acrylates d'isobutyle, de 2-éthylehexyle, de dodécyle, de stéaryle, de béhényle, et leurs mélanges.

Les monomères additionnels formant les éventuelles séquences ne comprenant ni monomère de formule (I), ni monomère siliconé, peuvent être choisis parmi, seuls ou en mélange, :

- les (méth)acrylates de formule $CH_2 = CHCOOR'_3$ ou $CH_2 = C(CH_3)COOR'_3$

dans lesquelles $R'_3$ représente un groupe alkyle linéaire ou ramifié, de 1 à 22 atomes de carbone, dans lequel se trouve (nt) éventuellement intercalé(s) un ou plusieurs hétéroatomes;

- les monomères à insaturation(s) éthylénique(s) comprenant au moins une fonction acide carboxylique, ou anhydride;
- (iv) les composés vinyliques de formules :
  $CH_2$=CH-$R_9$, $CH_2$=CH-$CH_2$-$R_9$ ou $CH_2$=C($CH_3$)-$CH_2$-$R_9$.
  En particulier, on peut citer l'acide acrylique, l'acide méthacrylique, et les (méth)acrylates d'isobutyle, de 2-éthylehexyle, de dodécyle, de stéaryle, de béhényle, le styrène et l'acétate de vinyle, ainsi que leurs mélanges.

Au final, les monomères siliconés, seuls ou en mélange, peuvent être présents à raison de 1 à 50% en poids, notamment à raison de 2 à 40% en poids, voire de 2,5 à 35% en poids, par rapport au poids total du polymère.

Les monomères de formule (I), seuls ou en mélange, peuvent être présents en une quantité de 20 à 80% en poids, notamment 25 à 70% en poids, encore mieux 30 à 60% en poids, par rapport au poids total du polymère.

De préférence, le polymère selon l'invention comprend une séquence de Tg supérieure ou égale à 20°C, par exemple comprise entre 20°C et 160°C, notamment comprise entre 30°C et 140°C, voire comprise entre 40°C et 120°C, préférentiellement comprise entre 50°C et 110°C, et qui est de préférence constituée des monomères carbonés (non

siliconés).

Il comprend également de préférence une séquence dont la Tg est inférieure à 20°C, par exemple comprise entre -150°C et 20°C exclu, notamment comprise entre -100°C et 10°C, voire entre -85°C et 0°C, préférentiellement comprise entre -70°C et -5°C, et qui comprend de préférence les monomères siliconés.

La séquence ayant une Tg supérieure ou égale à 20°C peut notamment comprendre, en totalité ou en partie, des monomères dont les homopolymères ont une Tg supérieure ou égale à 20°C. Elle peut également comprendre des monomères ayant une Tg en dehors de cette gamme. Ces monomères et leur concentration seront choisis de manière adéquate par l'homme du métier, notamment sur la base de la loi de Fox, pour obtenir une séquence de Tg souhaitée. Parmi ces monomères ayant une Tg supérieure ou égale à 20°C, on peut citer, seuls ou en mélange :

- les méthacrylates de formule : $CH_2 = C(CH_3)\text{-}COOR_1$

dans laquelle $R_1$ représente un groupe alkyle non substitué, linéaire ou ramifié, contenant de 1 à 4 atomes de carbone, tel qu'un groupe méthyle, éthyle, propyle ou isobutyle; ou bien $R_1$ représente un groupe cycloalkyle $C_4$ à $C_{12}$, notamment isobornyle;

- les acrylates de formule : $CH_2 = CH\text{-}COOR_2$ dans laquelle $R_2$ représente un groupe tertiobutyle ou bien un groupe cycloalkyle $C_4$ à $C_{12}$, notamment isobornyle;
- les (méth)acrylamides de formule : $CH_2 = CR'\text{-}CO\text{-}NR^7R^8$
  avec R' représentant H ou $CH_3$, et $R_7$ et $R_8$, identiques ou différents, représentent un atome d'hydrogène ou un groupe alkyle en $C_1$ à $C_{12}$ linéaire ou ramifié, tel qu'un groupe n-butyle, t-butyle, isopropyle, isohexyle, isooctyle, ou isononyle; ou encore $R_7$ représente H et $R_8$ représente un groupement 1,1-diméthyl-3-oxobutyle,
- l'acide méthacrylique et l'acide acrylique.
  Parmi les monomères dont les homopolymères ont une température de transition vitreuse Tg supérieure ou égale à 20°C plus particulièrement préférés, on peut citer le méthacrylate de méthyle, le méthacrylate d'éthyle, le méthacrylate d'isobutyle, le (méth)acrylate de tertio-butyle, l'acide (méth)acrylique, le (méth)acrylate d'isobornyle, le N-butylacrylamide, le N-t-butylacrylamide, le N-isopropylacrylamide, le N,N-diméthylacrylamide, le N,N-dibutylacrylamide et leurs mélanges.

La séquence ayant une Tg inférieure à 20°C peut notamment comprendre, en totalité ou en partie, des monomères dont les homopolymères ont une Tg inférieure à 20°C. Elle peut également comprendre des monomères ayant une Tg en dehors de cette gamme. Ces monomères et leur concentration seront choisis de manière adéquate par l'homme du métier, notamment sur la base de la loi de Fox, pour obtenir une séquence de Tg souhaitée.
Parmi ces monomères ayant une Tg inférieure à 20°C, on peut citer, seuls ou en mélange :

- les acrylates de formule $CH_2 = CHCOOR_3$,
  $R_3$ représentant un groupe alkyle non substitué en $C_1$ à $C_{12}$, linéaire ou ramifié, à l'exception du groupe tertiobutyle, dans lequel se trouve(nt) éventuellement intercalé(s) un ou plusieurs hétéroatomes choisis parmi O, N, S,
- les méthacrylates de formule $CH_2 = C(CH_3)\text{-}COOR_4$,
  $R_4$ représentant un groupe alkyle non substitué en $C_6$ à $C_{12}$ linéaire ou ramifié,

dans lequel se trouve(nt) éventuellement intercalé(s) un ou plusieurs hétéroatomes choisis parmi O, N et S;

- les esters vinyliques de formule $R_5\text{-}CO\text{-}O\text{-}CH{=}CH_2$ où $R_5$ représente un groupe alkyle en $C_4$ à $C_{12}$ linéaire ou ramifié ;
- les ($C_4$-$C_{12}$alkyl)-vinyléthers, notamment le méthylvinyléther et l'éthylvinyléther;
- les N-($C_4$-$C_{12}$ alkyl)acrylamides, tels que le N-octylacrylamide,
- et leurs mélanges.
  Parmi ces monomères de Tg inférieure à 20°C, on peut citer plus particulièrement l'acrylate de méthyle, l'acrylate d'éthyle, l'acrylate d'isobutyle, le (méth)acrylate d'éthyl-2-hexyle et leurs mélanges.

Dans un mode de réalisation préféré, tous les monomères autres que les monomères siliconés sont choisis parmi les esters d'acide (méth)acrylique et l'acide (méth)acrylique.

La masse molaire moyenne en poids (Mw) du polymère selon l'invention est de préférence comprise entre 25 000 et 1 000 000, mieux entre 30 000 et 750 000, voire entre 40 000 et 500 000, et préférentiellement entre 50 000 et 250 000.
On détermine les masses molaires moyennes en poids (Mw) et en nombre (Mn) par chromatographie liquide par perméation de gel (solvant THF, courbe d'étalonnage établie avec des étalons de polystyrène linéaire, détecteur réfractométrique et UV).

De préférence, l'indice de polydispersité du polymère selon l'invention est supérieur à 2, par exemple allant de 2 à 9, de préférence supérieur ou égal à 2,5, par exemple allant de 2,5 à 8, et mieux allant de 2,8 à 7. L'indice de polydispersité lp du polymère est égal au rapport de la masse moyenne en poids Mw sur la masse moyenne en nombre Mn.

Le polymère selon l'invention peut être obtenu par polymérisation radicalaire en solution selon le procédé de préparation suivant :

- on peut introduire une partie du solvant de polymérisation dans un réacteur adapté, on chauffe jusqu'à atteindre la température adéquate pour la polymérisation (typiquement entre 60 et 120°C),
- une fois cette température atteinte, on peut ajouter les monomères constitutifs de la première séquence, en présence d'une partie de l'initiateur de polymérisation,
- au bout d'un temps T correspondant à un taux de conversion maximum de 90% de préférence, on peut introduire les monomères constitutifs de la deuxième séquence et l'autre partie de l'initiateur,
- on laisse réagir le mélange pendant un temps T' (allant notamment de 3 à 6 heures) au bout duquel le mélange est ramené à température ambiante (25°C), de manière à obtenir le polymère en solution dans le solvant de polymérisation.

Par solvant de polymérisation, on entend un solvant, ou un mélange de solvants, notamment choisi parmi l'acétate d'éthyle, l'acétate de butyle, les alcools en C1-C6 tels que l'isopropanol, l'éthanol, les alcanes aliphatiques tels que l'isododécane et leurs mélanges. De préférence, le solvant de polymérisation est un mélange d'acétate de butyle et d'isopropanol ou est l'isododécane.

De préférence, le polymère selon l'invention n'est pas hydrosoluble, c'est à dire que le polymère n'est pas soluble dans l'eau ou dans un mélange d'eau et de monoalcools linéaires ou ramifiés ayant de 2 à 5 atomes de carbone comme l'éthanol, l'isopropanol ou le n-propanol, sans modification de pH, à une teneur en matière active d'au moins 1 % en poids, à température ambiante (25°C).

L'invention concerne également des compositions, notamment cosmétiques ou pharmaceutiques, comprenant au moins un polymère tel que décrit ci-dessus, dans un milieu physiologiquement, notamment cosmétiquement ou pharmaceutiquement, acceptable.

Les polymères selon l'invention peuvent être présents, seuls ou en mélange, dans les compositions selon l'invention en une quantité de 0,01 à 50% en poids, de préférence 0,05 à 40% en poids, notamment 0,1 à 30% en poids, voire 0,5 à 20% en poids, et encore mieux 1 à 15% en poids, préférentiellement 1,5 à 12% en poids, par rapport au poids total de la composition.

Ils peuvent être présents dans la composition sous forme solubilisée ou sous forme de dispersion, par exemple dans une huile ou un solvant organique carboné, tel que l'isododécane.

Avantageusement, les polymères selon l'invention sont solubles ou dispersibles dans au moins une des phases de la composition qui les comprend, à une teneur d'au moins 1% en poids, à pression et température ambiantes (25°C, 1 atm). Ils sont avantageusement solubles dans l'isododécane à une teneur d'au moins 1% en poids, notamment d'au moins 10% en poids, à pression et température ambiantes.

Les compositions cosmétiques ou pharmaceutiques selon l'invention comprennent, outre lesdits polymères, un milieu physiologiquement acceptable, notamment cosmétiquement ou pharmaceutiquement, acceptable, c'est-à-dire un milieu compatible avec les matières kératiniques telles que la peau du visage ou du corps, les cheveux, les cils, les sourcils et les ongles.

La composition peut ainsi comprendre, un milieu hydrophile comprenant de l'eau ou un mélange d'eau et de solvant(s) organique(s) hydrophile(s) comme les alcools et notamment les monoalcools inférieurs linéaires ou ramifiés ayant de 2 à 5 atomes de carbone comme l'éthanol, l'isopropanol ou le n-propanol, et les polyols comme la glycérine, la diglycérine, le propylène glycol, le sorbitol, le pentylène glycol, et les polyéthylène glycols, ou bien encore des éthers en $C_2$ et des aldéhydes en $C_2$-$C_4$ hydrophiles.

L'eau ou le mélange d'eau et de solvants organiques hydrophiles peut être présent dans la composition selon l'invention en une teneur allant de 0,1% à 99% en poids, par rapport au poids total de la composition, et de préférence de 10% à 80% en poids.

La composition peut également être anhydre.

La composition peut également comprendre une phase grasse qui peut comprendre des corps gras liquides à température ambiante (25°C en général) et/ou des corps gras solides à température ambiante tels que les cires, les corps gras pâteux, les gommes et leurs mélanges. Ces corps gras peuvent être d'origine animale, végétale, minérale ou synthétique. Cette phase grasse peut, en outre, contenir des solvants organiques lipophiles.

Comme corps gras liquides à température ambiante, appelés souvent huiles, utilisables dans l'invention, on peut citer : les huiles hydrocarbonées d'origine animale telles que le perhydrosqualène; les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raisin, de sésame, d'abricot, de

macadamia, de ricin, d'avocat, les triglycérides des acides caprylique/caprique, l'huile de jojoba, de beurre de karité ; les hydrocarbures linéaires ou ramifiés, d'origine minérale ou synthétique tels que les huiles de paraffine et leurs dérivés, la vaseline, les polydécènes, le polyisobutène hydrogéné tel que le parléam; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras; des esters de polyol comme le dioctanoate de propylène glycol, le diheptanoate de néopentylglycol, le diisononanoate de diéthylèneglycol ; et les esters du pentaérythritol ; des alcools gras ayant de 12 à 26 atomes de carbone comme l'octyldodécanol, le 2-butyloctanol, le 2-hexyldécanol, le 2-undécylpentadécanol, l'alcool oléique; les huiles fluorées partiellement hydrocarbonées et/ou siliconées; les huiles siliconées comme les polyméthylsiloxanes (PDMS) volatiles ou non, linéaires ou cycliques, liquides ou pâteux à température ambiante comme les cyclométhicones, les diméthicones, comportant éventuellement un groupement phényle, comme les phényl triméthicones, les phényltriméthylsiloxydiphényl siloxanes, les diphénylméthyldiméthyltrisiloxanes, les diphényl diméthicones, les phényl diméthicones, les polyméthylphényl siloxanes ; leurs mélanges. Ces huiles peuvent être présentes en une teneur allant de 0,01 à 90%, et mieux de 0,1 à 85% en poids, par rapport au poids total de la composition.

La composition selon l'invention peut également comprendre un ou plusieurs solvants organiques, physiologiquement acceptables.

Ces solvants peuvent être généralement présents en une teneur allant de 0,1 à 90%, de préférence de 0,5 à 85%, de préférence encore de 10 à 80% en poids, par rapport au poids total de la composition, et mieux de 30 à 50 %.

On peut notamment citer, outre les solvants organiques hydrophiles cités plus haut, les cétones liquides à température ambiante tels que méthyléthylcétone, méthylisobutylcétone, diisobutylcétone, l'isophorone, la cyclohexanone, l'acétone; les éthers de propylène glycol liquides à température ambiante tels que le monométhyléther de propylène glycol, l'acétate de monométhyl éther de propylène glycol, le mono n-butyl éther de dipropylène glycol; les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle; les éthers liquides à 25°C tels que le diéthyléther, le diméthyléther ou le dichlorodiéthyléther; les alcanes liquides à 25°C tels que le décane, l'heptane, le dodécane, l'isododécane, le cyclohexane; les composés cycliques aromatiques liquides à 25°C tels que le toluène et le xylène; les aldéhydes liquides à 25°C tels que le benzaldéhyde, l'acétaldéhyde et leurs mélanges.

Par cire au sens de la présente invention, on entend un composé lipophile, solide à température ambiante (25°C), à changement d'état solide/liquide réversible, ayant un point de fusion supérieur ou égal à 25°C pouvant aller jusqu'à 120°C. En portant la cire à l'état liquide (fusion), il est possible de la rendre miscible aux huiles éventuellement présentes et de former un mélange homogène microscopiquement, mais en ramenant la température du mélange à la température ambiante, on obtient une recristallisation de la cire dans les huiles du mélange. Le point de fusion de la cire peut être mesuré à l'aide d'un calorimètre à balayage différentiel (D.S.C.), par exemple le calorimètre vendu sous la dénomination DSC 30 par la société METLER.

Les cires peuvent être hydrocarbonées, fluorées et/ou siliconées et être d'origine végétale, minérale, animale et/ou synthétique. En particulier, les cires présentent un point de fusion supérieur à 30°C et mieux supérieur à 45°C. Comme cire utilisable dans la composition de l'invention, on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxydiméthicone ayant de 16 à 45 atomes de carbone.

Les gommes sont généralement des polydiméthylsiloxanes (PDMS) à haut poids moléculaire ou des gommes de cellulose ou des polysaccharides et les corps pâteux sont généralement des composés hydrocarbonés comme les lanolines et leurs dérivés ou encore des PDMS.

Par corps gras pâteux, on entend un produit visqueux contenant une fraction liquide et une fraction solide. On entend notamment des corps gras ayant un point de fusion allant de 20 à 55°C, de préférence 25 à 45°C, et/ou une viscosité à 40°C allant de 0,1 à 40 Pa.s (1 à 400 poises), de préférence 0,5 à 25 Pa.s, mesurée au Contraves TV ou Rhéomat 80. L'homme du métier peut choisir le mobile permettant de mesurer la viscosité, parmi les mobiles MS-r3 et MS-r4, sur la base de ses connaissances générales, de manière à pouvoir réaliser la mesure de la viscosité du composé pâteux testé.

Les valeurs de point de fusion correspondent, selon l'invention, au pic de fusion mesurée par la méthode "Differential Scanning Calorimetry" avec une montée en température de 5 ou 10 °C/min.

De préférence, ces corps gras sont des composés hydrocarbonés (contenant principalement des atomes de carbone et d'hydrogène et éventuellement des groupements ester), éventuellement de type polymérique; ils peuvent également être choisis parmi les composés siliconés et/ou fluorés; ils peuvent aussi se présenter sous forme d'un mélange de composés hydrocarbonés et/ou siliconés et/ou fluorés. Dans le cas d'un mélange de différents corps gras pâteux, on utilise de préférence les composés pâteux hydrocarbonés en proportion majoritaire.

Parmi les composés pâteux susceptibles d'être utilisés dans la composition selon l'invention, on peut citer les lanolines et les dérivés de lanoline comme les lanolines acétylées ou les lanolines oxypropylènées ou le lanolate d'isopropyle;

des esters d'acides ou d'alcools gras, notamment ceux ayant 20 à 65 atomes de carbone comme le citrate de triisostéaryle ou de cétyle; le propionate d'arachidyle; le polylaurate de vinyle; les esters du cholestérol comme les triglycérides d'origine végétale tels que les huiles végétales hydrogénées, les polyesters visqueux comme l'acide poly(12-hydroxystéarique) et leurs mélanges. Comme triglycérides d'origine végétale, on peut utiliser les dérivés d'huile de ricin hydrogénée.

On peut aussi citer les corps gras pâteux siliconés tels que les polydiméthylsiloxanes (PDMS) ayant des chaînes pendantes du type alkyle ou alcoxy ayant de 8 à 24 atomes de carbone, comme les stéaryl diméthicones.

La nature et la quantité des corps solides sont fonction des propriétés mécaniques et des textures recherchées. A titre indicatif, la composition peut contenir de 0,1 à 50% en poids de cires, par rapport au poids total de la composition et mieux de 1 à 30% en poids.

La composition selon l'invention peut en outre comprendre, dans une phase particulaire, des pigments et/ou des nacres et/ou des charges habituellement utilisés dans les compositions cosmétiques.

La composition peut également comprendre d'autres matières colorantes choisies parmi les colorants hydrosolubles et/ou les colorants liposolubles bien connus de l'homme du métier.

Par pigments, il faut comprendre des particules de toute forme, blanches ou colorées, minérales ou organiques, insolubles dans le milieu physiologique, destinées à colorer la composition.

Par charges, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage.

Par nacres, il faut comprendre des particules de toute forme irisées, notamment produites par certains mollusques dans leur coquille ou bien synthétisées.

Les pigments peuvent être présents dans la composition à raison de 0,01 à 25% en poids de la composition finale, et de préférence à raison de 3 à 10% en poids. Ils peuvent être blancs ou colorés, minéraux ou organiques,. On peut citer les oxydes de titane, de zirconium ou de cérium, ainsi que les oxydes de zinc, de fer ou de chrome, le bleu ferrique, l'hydrate de chrome, le noir de carbone, les outremers (polysulfures d'aluminosilicates), le pyrophosphate de manganèse et certaines poudres métalliques telles que celles d'argent ou d'aluminium. On peut encore citer les pigments D&C et les laques couramment employées pour conférer aux lèvres et à la peau un effet de maquillage, qui sont des sels de calcium, de baryum, d'aluminium, de strontium ou de zirconium.

Les nacres peuvent être présentes dans la composition à raison de 0,01 à 20% en poids, de préférence à un taux de l'ordre de 3 à 10% en poids. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.

Parmi les colorants, liposolubles ou hydrosolubles, qui peuvent être présents dans la composition, seul ou en mélange, à raison de 0,001 à 15% en poids, de préférence 0,01 à 5% en poids et notamment de 0,1 à 2% en poids, par rapport au poids total de la composition, on peut citer le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle, le bleu de méthylène, le carmin de cochenille, les colorants halogéno-acides, azoïques, anthraquinoniques, le sulfate de cuivre ou de fer, le brun Soudan, le rouge Soudan et le rocou, ainsi que le jus de betterave et le carotène.

La composition selon l'invention peut comprendre en outre en outre une ou plusieurs charges, notamment en une teneur allant de 0,01% à 50% en poids, par rapport au poids total de la composition, de préférence allant de 0,02% à 30% en poids. Les charges peuvent être minérales ou organiques de toute forme, plaquettaires, sphériques ou oblongues. On peut citer le talc, le mica, la silice, le kaolin, les poudres de polyamide (Nylon®), de poly-β-alanine et de polyéthylène, les poudres de polymères de tétrafluoroéthylène (Téflon®), la lauroyllysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Corning) et les microbilles de résine de silicone (Tospearls® de Toshiba, par exemple), les particules de polyorganosiloxanes élastomères, le carbonate de calcium précipité, le carbonate et l'hydrocarbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La composition peut comprendre en outre un polymère additionnel tel qu'un polymère filmogène. Selon la présente invention, on entend par "polymère filmogène", un polymère apte à former à lui seul ou en présence d'un agent auxiliaire de filmification, un film continu et adhérent sur un support, notamment sur les matières kératiniques. Parmi les polymères filmogènes susceptibles d'être utilisés dans la composition de la présente invention, on peut citer les polymères synthétiques, de type radicalaire ou de type polycondensat, les polymères d'origine naturelle et leurs mélanges, en particulier les polymères acryliques, les polyuréthanes, les polyesters, les polyamides, les polyurées, les polymères cellulosiques comme la nitrocellulose.

La composition selon l'invention peut également comprendre des ingrédients couramment utilisés en cosmétique, tels que les vitamines, les épaississants, les gélifiants, les oligo-éléments, les adoucissants, les séquestrants, les parfums, les agents alcalinisants ou acidifiants, les conservateurs, les filtres solaires, les tensioactifs, les anti-oxydants, les agents anti-chutes des cheveux, les agents anti-pelliculaires, les agents propulseurs, les céramides, les agents auxiliaires de filmification, ou leurs mélanges.
Bien entendu, l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

La composition selon l'invention peut se présenter sous la forme d'une suspension, une dispersion notamment d'huile dans de l'eau grâce à des vésicules; une solution aqueuse ou huileuse éventuellement épaissie voire gélifiée; une émulsion huile-dans-eau, eau-dans-huile, ou multiple; un gel ou une mousse; un gel huileux ou émulsionné; une dispersion de vésicules notamment lipidiques; une lotion biphase ou multiphase; un spray; une poudre libre, compacte ou coulée; une pâte anhydre. Cette composition peut avoir l'aspect d'une lotion, d'une crème, d'une pommade, d'une pâte souple, d'un onguent, d'une mousse, d'un solide coulé ou moulé et notamment en stick ou en coupelle, ou encore de solide compacté.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

La composition cosmétique selon l'invention peut se présenter sous la forme d'un produit de soin et/ou de maquillage de la peau du corps ou du visage, des lèvres, des ongles, des cils, des sourcils et/ou des cheveux, d'un produit solaire ou autobronzant, d'un produit capillaire pour le soin, le traitement, la mise en forme, le maquillage ou la coloration des cheveux.

Elle peut ainsi se présenter sous la forme d'une composition de maquillage, notamment un produit pour le teint tel qu'un fond de teint, un fard à joues ou à paupières; un produit pour les lèvres tel qu'un rouge à lèvres ou un soin des lèvres; un produit anti-cernes; un blush, un mascara, un eye-liner; un produit de maquillage des sourcils, un crayon à lèvres ou à yeux; un produit pour les ongles tel qu'un vernis à ongles ou un soin des ongles; un produit de maquillage du corps; un produit de maquillage des cheveux (mascara ou laque pour cheveux).
Elle peut également se présenter sous forme d'une composition de protection ou de soin de la peau du visage, du cou, des mains ou du corps, notamment une composition anti-rides, une composition hydratante ou traitante; une composition anti-solaire ou de bronzage artificiel.
Elle peut encore se présenter sous forme d'un produit capillaire, notamment pour la coloration, le maintien de la coiffure, la mise en forme des cheveux, le soin, le traitement ou le nettoyage des cheveux, telle que des shampooings, des gels, des lotions de mise en plis, des lotions pour le brushing, des compositions de fixation et de coiffage telles que les laques ou spray.

De préférence, la composition cosmétique selon l'invention se présente sous la forme d'un produit de maquillage, notamment d'un rouge à lèvres.

L'invention a également pour objet un procédé de traitement cosmétique, notamment de maquillage ou de soin des matières kératiniques, telles que la peau du corps ou du visage, des lèvres, des ongles, des cils, des sourcils et/ou des cheveux, comprenant l'application sur lesdites matières d'une composition cosmétique telle que définie précédemment.

L'invention est illustrée plus en détail dans les exemples suivants.

<u>Brillance mesurée au brillancemètre sur un dépôt sec de polymère</u>

La brillance peut être mesurée à l'aide d'un brillancemètre, de manière conventionnelle par la méthode suivante. Sur une carte de contraste de marque LENETA et de référence FORM 1A PENOPAC, on étale une couche de 50 μm d'épaisseur d'une solution du polymère à 50% dans l'isododécane à l'aide d'un étaleur automatique. La couche recouvre au moins le fond noir de la carte. On laisse sécher le dépôt 24 heures à une température de 25°C, puis on procède à la mesure de la brillance à 20° sur le fond noir à l'aide d'un brillancemètre de marque DR LANGE, REF03.
Une mesure à 20° supérieure à environ 50 donne une brillance jugée acceptable, et très satisfaisante si la mesure est supérieure à 60.

<u>Tenue à l'huile des polymères par mesure de l'aspect collant</u>

Elle est déterminée avec une goutte d'huile d'olive mise sur un film de polymère sec.
Un film de polymère est réalisé à partir d'une solution de polymère à 20% dans l'isododécane; 0,5 ml est étalé sur une plaque de verre de 2,5 x 7,5 cm et laissé sécher à température ambiante (25°C) pendant 24 heures. Ensuite, 1 ml d'huile d'olive est étalé sur le film de polymère.
Après 1 heure, l'excès d'huile est essuyé du film et l'aspect collant est évalué au toucher, par comparaison avec le

polymère comparatif.

Une note de '***' ou '3*' est donnée lorsque l'on décolle la plaque avec le doigt (très collant).
Une note de '0' signifie : pas de collant détecté.
Le collant traduit la sensibilité du polymère à l'huile d'olive. Plus le polymère est collant en présence d'huile, plus il est sensible à l'huile et donc plus le dépôt sera facilement altéré, par exemple lors des repas (en présence d'huile alimentaire) ou par le sébum. Il en résulte une moins bonne tenue du polymère sur la peau. Il en résulte aussi une diminution du confort : plus le film est collant, plus la composition est inconfortable à porter.

CI : contraintes internes

Un film de polymère est réalisé à partir d'une solution de polymère à 20% dans l'isododécane; on étale 30 microlitres sur une bande de nitrile de 6 x 1 cm et on laisse sécher à température ambiante (25°C) pendant 3 heures. On observe visuellement la déformation du support après séchage.
Une note de 0 signifie que le support ne s'est pas déformé lors du séchage.
Une note de +++ signifie que le support est presque plié (complètement déformé) après séchage.
Les contraintes internes (CI) reflètent le confort du point de vue mécanique: lorsqu'il n'y a pas de déformation du support lors du séchage, on peut penser qu'il n'y aura pas d'inconfort lors du séchage de la composition après son application sur la peau.

## Exemple 1

On introduit 300 g d'isododécane dans un réacteur de 1 litre, puis on augmente la température de façon à passer de la température ambiante (25°C) à 90 °C en 1 heure. On ajoute ensuite, à 90°C et en 1 heure, 105 g d'acrylate d'isobornyle, 105 g de méthacrylate d'isobornyle et 1,8 g de 2.5-bis(2-éthylhexanoylperoxy)-2.5-diméthylhexane (Trigonox® 141 d'Akzo Nobel). Le mélange est maintenu 1 h 30 à 90 °C.
On introduit ensuite au mélange précédent, toujours à 90°C et en 30 minutes, 82,5 g d'acrylate d'isobutyle, 7,5 g de MPS et 1,2 g de 2.5-bis(2-éthylhexanoylperoxy)-2.5-diméthylhexane. Le mélange est maintenu 3 heures à 90°C, puis l'ensemble est refroidi.
On obtient une solution à 50,8% de matière active en polymère dans l'isododécane. Ledit polymère comprend une 1ère séquence comprenant les (méth)acrylates d'isobornyle et une deuxième séquence comprenant le monomère siliconé et l'acrylate d'isobutyle.

## Exemple 2

On introduit 300 g d'isododécane dans un réacteur de 1 litre, puis on augmente la température de façon à passer de la température ambiante (25°C) à 90 °C en 1 heure. On ajoute ensuite, à 90°C et en 1 heure, 105 g d'acrylate d'isobornyle, 105 g de méthacrylate d'isobornyle et 1,8 g de 2.5-bis(2-éthylhexanoylperoxy)-2.5-diméthylhexane (Trigonox® 141 d'Akzo Nobel). Le mélange est maintenu 1 h 30 à 90°C.
On introduit ensuite au mélange précédent, toujours à 90°C et en 30 minutes, 82,5 g d'acrylate d'isobutyle, 7.5 g de MPTS et 1,2 g de 2,5-bis(2-éthylhexanoylperoxy)-2,5-diméthylhexane. Le mélange est maintenu 3 heures à 90°C, puis l'ensemble est refroidi.
On obtient une solution à 50,8% de matière active en polymère dans l'isododécane. Ledit polymère comprend une 1ère séquence comprenant les (méth)acrylates d'isobornyle et une deuxième séquence comprenant le monomère siliconé et l'acrylate d'isobutyle.

## Exemple 3

On introduit 300 g d'isododécane dans un réacteur de 1 litre, puis on augmente la température de façon à passer de la température ambiante (25°C) à 90°C en 1 heure. On ajoute ensuite, à 90°C et en 1 heure, 97,5 g d'acrylate d'isobornyle, 105 g de méthacrylate d'isobornyle, 7,7 g de MPTS et 1,8 g de 2.5-bis(2-éthylhexanoylperoxy)-2.5-diméthylhexane (Trigonox® 141 d'Akzo Nobel). Le mélange est maintenu 1 h 30 à 90°C.
On introduit ensuite au mélange précédent, toujours à 90°C et en 30 minutes, 90 g d'acrylate d'isobutyle et 1,2 g de 2,5-bis(2-éthylhexanoylperoxy)-2,5-diméthylhexane. Le mélange est maintenu 3 heures à 90°C, puis l'ensemble est refroidi.
On obtient une solution à 49,2% de matière active en polymère dans l'isododécane. Ledit polymère comprend une 1ère séquence comprenant les (méth)acrylates d'isobornyle et le monomère siliconé et une deuxième séquence comprenant l'acrylate d'isobutyle.

**Exemple comparatif (hors invention)**

On introduit 100 g d'isododécane dans un réacteur de 1 litre, puis on augmente la température de façon à passer de la température ambiante (25°C) à 90°C en 1 heure. On ajoute ensuite, à 90°C et en 1 heure, 105 g d'acrylate d'isobornyle, 105 g de méthacrylate d'isobornyle, 110 g d'isododécane et 1,8 g de 2,5-bis(2-éthylhexanoylperoxy)-2,5-diméthylhexane (Trigonox® 141 d'Akzo Nobel). Le mélange est maintenu 1 h 30 à 90°C.
On introduit ensuite au mélange précédent, toujours à 90°C et en 30 minutes, 90 g d'acrylate d'isobutyle, 90 g d'isododécane et 1,2 g de 2,5-bis(2-éthylhexanoylperoxy)-2,5-diméthylhexane. Le mélange est maintenu 3 heures à 90°C, puis l'ensemble est refroidi.
On obtient une solution à 50% de matière active en polymère dans l'isododécane. Ledit polymère comprend une 1ère séquence comprenant les (méth)acrylates d'isobornyle et une deuxième séquence comprenant l'acrylate d'isobutyle.

**Exemple 4**

On caractérise les films obtenus à partir des polymères des exemples 1 à 3, ainsi qu'avec le polymère comparatif préparé ci-dessus :

- Film : on observe visuellement le caractère cassant d'un film seul de polymère.
- On détermine également, selon les méthodes indiquées plus haut, le collant (tenue à l'huile d'olive) et les contraintes internes du polymère.

On obtient les résultats suivants :

| | Exemple 1 | Exemple 2 | Exemple 3 | Comparatif |
|---|---|---|---|---|
| % A. Isob. | 35 | 35 | 32.5 | 35 |
| % M. Isob. | 35 | 35 | 35 | 35 |
| % M PTS | -- | 2.5 | 2.5 | -- |
| % MPS | 2.5 | -- | -- | -- |
| % A. Ibut. | 27.5 | 27.5 | 30 | 30 |
| Mw (g/mol) | 92 800 | 109 800 | 128 900 | 100300 |
| Ip | 3,97 | 3,26 | 3,98 | 4,40 |
| ES (%) | 50,8 | 50,8 | 49,2 | 50,0 |
| Film seul (observation visuelle) | légèrement cassant | légèrement cassant | à peine cassant | cassant |
| Brillance à 20° | 76,2 | 66,1 | 60,8 | 71,3 |
| Collant | 0 | 0 | +++ | +++ |
| Contraintes internes | ++ | ++ | ++ | +++ |
| A. Isob. : acrylate d'isobornyle<br>M. Isob. : méthacrylate d'isobornyle<br>MPTS : méthacryloxypropyltris(trimethylsiloxy)silane<br>MPS : méthacryloxypropyltrimethoxysilane<br>A. Ibut : acrylate d'isobutyle<br>ES : extrait sec | | | | |

**Exemple 5**

On prépare un stick de rouge à lèvres comprenant (% en poids):

- Cire de polyéthylène 15%
- Solution du polymère de l'exemple 1
  à 50,8% matière sèche dans l'isododécane 20%
- Polyisobutène hydrogéné (Parléam de Nippon Oil Fats) 25%

- Pigments 10%
- Isododécane qsp 100%

La composition obtenue après application sur les lèvres présente de bonnes propriétés cosmétiques.

## Revendications

1. Polymère séquencé comprenant au moins une première séquence et au moins une deuxième séquence, dans lequel :

   - au moins l'une des séquences, dite première séquence, comprend de 0,5 à 35% en poids, par rapport au poids de ladite séquence, d'au moins un monomère siliconé choisi parmi, seul ou en mélange, les monomères suivants :
   - (i) les monomères éthyléniques dont le groupement ester contient des silanes et/ou des siloxanes, de formule :

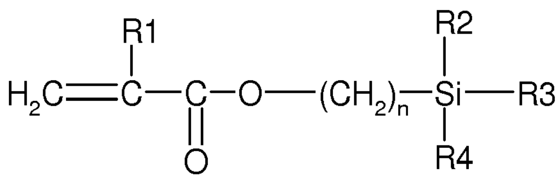

   dans laquelle :

   - R1= H ou méthyle,
   - R2, R3, R4, identiques ou différents, représentent des groupes alkyles en C1-C6 ou le groupe $-OSi(R5)_3$ avec R5= méthyle ou éthyle; de préférence R2, R3 et/ou R4 représentent, indépendamment l'un de l'autre, $-OSi(Me)_3$ et/ou méthyle;
   - n est un nombre entier de 1 à 10, de préférence égal à 1 ou 3.
   - (ii) les macromonomères PDMS, ou polydiméthylsiloxanes à groupement terminal monoacryloyloxy ou monométhacryloyloxy, de formule suivante

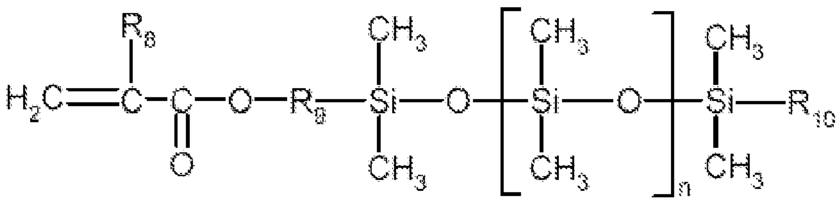

   dans laquelle :

   - R8 désigne un atome d'hydrogène ou un groupement méthyle; de préférence méthyle;
   - R9 désigne un groupe hydrocarboné divalent, linéaire ou ramifié, de préférence linéaire, ayant de 1 à 10 atomes de carbone et contenant éventuellement une ou deux liaisons éther -O- ; de préférence éthylène, propylène ou butylène;
   - R10 désigne un groupe alkyle linéaire ou ramifié, ayant de 1 à 10 atomes de carbone, notamment de 2 à 8 atomes de carbone; de préférence méthyle, éthyle, propyle, butyle ou pentyle;
   - n désigne un nombre entier allant de 1 à 300, de préférence allant de 3 à 200, et préférentiellement allant de 5 à 100.
   - (iii) les monomères éthyléniques dont le groupement ester contient des carboxysilanes dendrimères de formule :

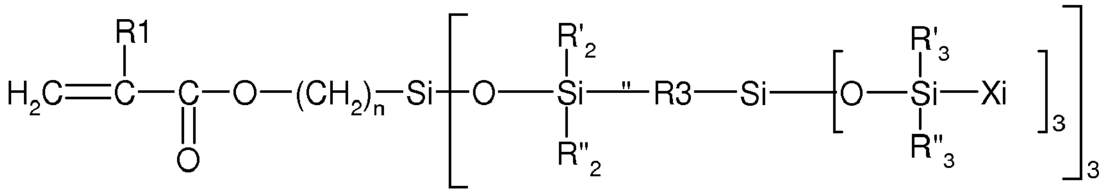

dans laquelle :

- R1= H ou méthyle;
- n est un nombre entier de 1 à 10, de préférence égal à 1, 2, 3 ou 4;
- R'2, R"2, R'3 et R"3, identiques ou différents, représentent un groupe alkyle en C1 à C10, de préférence méthyle ou éthyle;
- R3 est un groupe divalent alkylène en C2 à C10, de préférence en C2 ou C3;
- i est un entier compris entre 1 et 10, notamment i = 1, 2 ou 3;
- quand i = 2 à 10, X(i), identique ou différent, représente un groupe -R4-Si-[O-(R'3)(R"3)-X(i-1)]$_3$, avec R4, identique ou différent, représentant un groupe divalent alkylène en C2 à C10, de préférence en C2 ou C3;
- et X(1) représente H ou un groupe alkyle en C1-C10, notamment méthyle ou éthyle;
- (iv) les monomères éthyléniques de type POSS ou POS de structure :

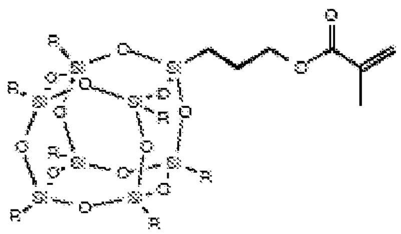

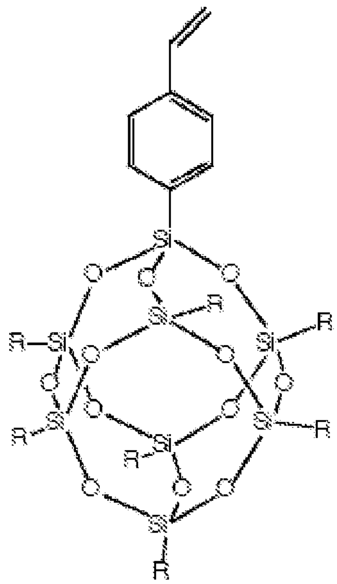

dans lesquelles R, identique ou différent, est un groupe alkyle linéaire en C1 à C10, de préférence méthyle, ou cyclique en C3 à C12, de préférence en C5; et

- au moins une séquence, identique ou différente de ladite première séquence, comprend de 0,5 à 100% en poids, par rapport au poids de ladite séquence, d'au moins un monomère choisi parmi, seul ou en mélange, les monomères de formule (I) : $CH_2$=C(R1)-COOR2

dans laquelle R1 est H ou $CH_3$ et R2 représente un groupe cycloalkyle en C4 à C12, notamment en C6-C10.

**2.** Polymère selon la revendication 1, dans lequel les monomères siliconés sont choisis parmi, seuls ou en mélange,:

- le (méth)acryloxypropyltris(trimethylsiloxy)silane, le (méth)acryloxypropylbis-(trimethylsiloxy)méthylsilane, le (méth)acryloxyméthyltris(trimethylsiloxy)silane, le (méth)acryloxyméthylbis(trimethylsiloxy)méthylsilane; le (méth)acryloxypropyltrimethoxysilane;

- les monomères suivants, dans lesquels R1 représente H ou méthyle :

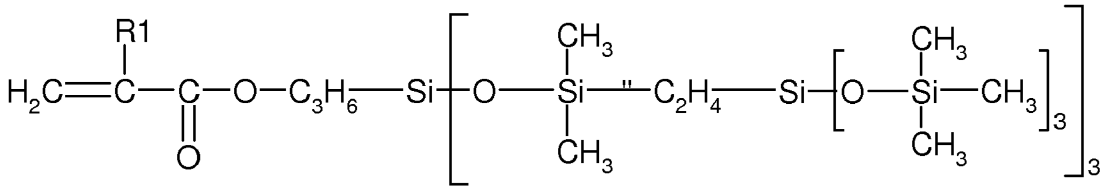

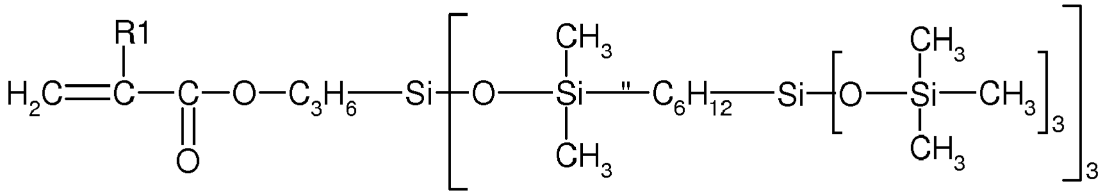

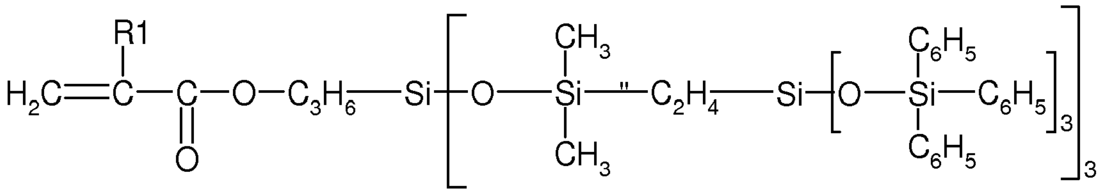

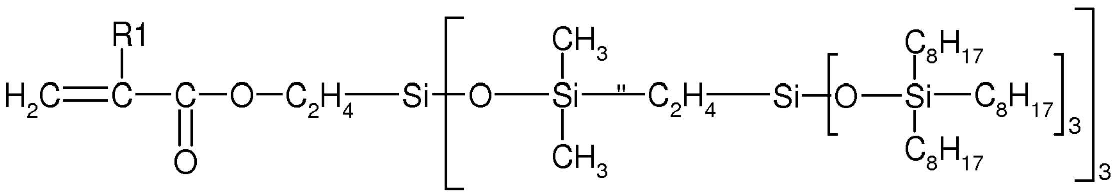

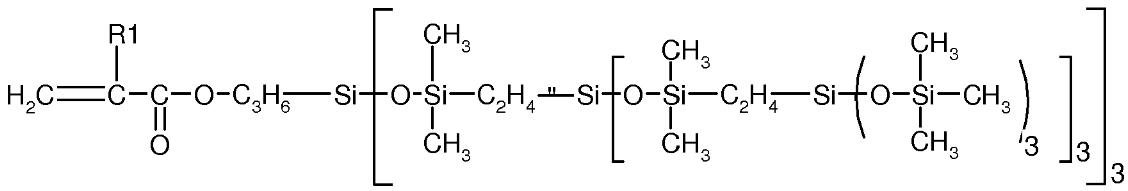

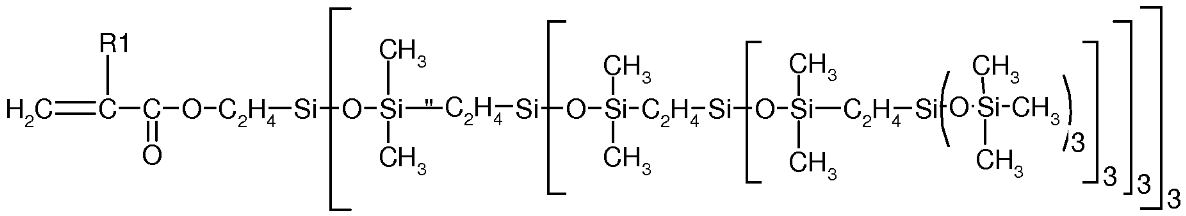

3. Polymère selon l'une des revendications précédentes, dans lequel les monomères siliconés représentent 0,5% à 35% en poids, notamment 1 à 30% en poids, voire 1,5 à 25% en poids, encore mieux 2 à 20% en poids, et préférentiellement 3 à 10% en poids, du poids total de la séquence les comprenant.

4. Polymère selon l'une des revendications précédentes, dans lequel les monomères de formule (I) sont choisis parmi le méthacrylate d'isobornyle, l'acrylate d'isobornyle, l'acrylate et le méthacrylate de cyclohexyle, l'acrylate et le méthacrylate de t-butylcyclohexyle, et leurs mélanges.

5. Polymère selon l'une des revendications précédentes, dans lequel les monomères siliconés et les monomères de formule (I) sont présents dans la même séquence.

6. Polymère selon la revendication 5, dans lequel les monomères de formule (I) représentent 0,5 à 99,5% en poids, notamment 5 à 99% en poids, voire 20 à 90% en poids, encore mieux 40 à 80% en poids, préférentiellement 50 à 70% en poids, du poids total de la séquence les comprenant.

7. Polymère selon l'une des revendications 1 à 4, dans lequel les monomères siliconés et les monomères de formule (I) sont présents dans des séquences différentes.

8. Polymère selon la revendication 7, dans lequel les monomères de formule (I) représentent 0,5 à 100% en poids, notamment 5 à 99% en poids, voire 20 à 90% en poids, encore mieux 40 à 80% en poids, préférentiellement 50 à 70% en poids, du poids total de la séquence les comprenant.

9. Polymère selon l'une des revendications précédentes, comprenant en outre un ou plusieurs monomères additionnels, choisis parmi, seul ou en mélange, :

- (i) les hydrocarbures éthyléniques ayant 2 à 10 carbones, tels que l'éthylène, l'isoprène, ou le butadiène ;
- (ii) les (méth)acrylates de formule $CH_2$ = CHCOOR'$_3$ ou $CH_2$ = C($CH_3$)COOR'$_3$

dans lesquelles R'$_3$ représente :

- un groupe alkyle linéaire ou ramifié, de 1 à 22 atomes de carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi les groupes hydroxyle ou les atomes d'halogène (Cl, Br, I et F);
- un groupe aryle en $C_3$ à $C_{20}$ tel que le groupe phényle,
- un groupe aralkyle en $C_4$ à $C_{30}$ (groupe alkyle en $C_1$ à $C_8$) tel que 2-phényl-éthyle, t-butylbenzyle ou benzyle,
- un groupe hétérocyclique de 4 à 12 chaînons contenant un ou plusieurs hétéroatomes choisis parmi O, N, et S, le cycle étant aromatique ou non,
- un groupe hétérocycloalkyle (alkyle en C1-C4), tel que furfurylméthyle ou tétrahydrofurfurylméthyle,
lesdits groupes aryle, aralkyle, hétérocyclique ou hétérocycloalkyle pouvant être éventuellement substitué par un ou plusieurs substituants choisis parmi les groupes hydroxyles, les atomes d'halogène, et les groupes alkyles en C1-4, linéaires ou ramifiés dans lesquels se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi les groupes hydroxyle ou les atomes d'halogène (Cl, Br, I et F),
- R'$_3$ peut également être un groupe -($C_2H_4O$)$_m$-R", avec m = 5 à 150 et R" = H ou alkyle de $C_1$ à $C_{30}$, par exemple -POE-méthyle ou -POE-béhényle;
- (iii) les (méth)acrylamides de formule :

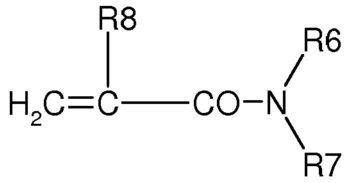

dans laquelle $R_8$ désigne H ou méthyle; et $R_7$ et $R_6$ identiques ou différents représentent :

- un atome d'hydrogène; ou
- un groupe alkyle linéaire ou ramifié, de 1 à 22 atomes de carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi les groupes hydroxyle, les atomes d'halogène (Cl, Br, I et F);
- un groupe cycloalkyle en $C_3$ à $C_{12}$, tel que le groupe isobornyle,
- un groupe aryle en $C_3$ à $C_{20}$ tel que le groupe phényle,
- un groupe aralkyle en $C_4$ à $C_{30}$ (groupe alkyle en $C_1$ à $C_8$) tel que 2-phényl-éthyle, t-butylbenzyle ou benzyle,
- un groupe hétérocyclique de 4 à 12 chaînons contenant un ou plusieurs hétéroatomes choisis parmi O, N, et S, le cycle étant aromatique ou non,
- un groupe hétérocycloalkyle (alkyle en C1-C4), tel que furfurylméthyle ou tétrahydrofurfurylméthyle,

lesdits groupes cycloalkyle, aryle, aralkyle, hétérocyclique ou hétérocycloalkyle pouvant être éventuellement substitué par un ou plusieurs substituants choisis parmi les groupes hydroxyles, les atomes d'halogène, et les groupes alkyles en C1-C4, linéaires ou ramifiés dans lesquels se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi les groupes hydroxyle et les atomes d'halogène (Cl, Br, I et F).

- (iv) les composés vinyliques de formules :

$CH_2$=CH-$R_9$, $CH_2$=CH-$CH_2$-$R_9$ ou $CH_2$=C($CH_3$)-$CH_2$-$R_9$

dans lesquelles $R_9$ est un groupe hydroxyle, halogène (Cl ou F), $NH_2$, $OR_{14}$ où $R_{14}$ représente un groupe phényle ou un groupe alkyle en $C_1$ à $C_{12}$ ; acétamide ($NHCOCH_3$); un groupe $OCOR_{15}$ où $R_{15}$ représente un groupe alkyle de 2 à 12 carbones, linéaire ou ramifié; ou un groupe choisi parmi :

- un groupe alkyle linéaire ou ramifié, de 1 à 22 atomes de carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi les groupes hydroxyle, les atomes d'halogène (Cl, Br, I et F),
- un groupe cycloalkyle en $C_3$ à $C_{12}$ tel que isobornyle, cyclohexyle,
- un groupe aryle en $C_3$ à $C_{20}$ tel que phényle,
- un groupe aralkyle en $C_4$ à $C_{30}$ (groupe alkyle en $C_1$ à $C_8$) tel que 2-phényléthyle ; benzyle,
- un groupe hétérocyclique de 4 à 12 chaînons contenant un ou plusieurs hétéroatomes choisis parmi O, N, et S, le cycle étant aromatique ou non,
- un groupe hétérocycloalkyle (alkyle en C1-C4), tel que furfurylméthyle ou tétrahydrofurfurylméthyle,

lesdits groupes cycloalkyle, aryle, aralkyle, hétérocyclique ou hétérocycloalkyle pouvant être éventuellement substitué par un ou plusieurs substituants choisis parmi les groupes hydroxyles, les atomes d'halogène, et les groupes alkyles de 1 à 4 C linéaires ou ramifiés dans lesquels se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi les groupes hydroxyle les atomes d'halogène (Cl, Br, I et F);

- (v) les monomères à insaturation(s) éthylénique(s) comprenant au moins une fonction acide carboxylique, phosphorique ou sulfonique, ou anhydride, comme par exemple l'acide acrylique, l'acide méthacrylique, l'acide crotonique, l'anhydride maléique, l'acide itaconique, l'acide fumarique, l'acide maléique, l'acide acrylamidopropanesulfonique, l'acide vinylbenzoïque, l'acide vinylphosphorique et les sels de ceux-ci,
- (vi) les monomères à insaturation(s) éthylénique(s) comprenant au moins une fonction amine tertiaire comme la 2-vinylpyridine, la 4-vinylpyridine, le méthacrylate de diméthylaminoéthyle, le méthacrylate de diéthylaminoéthyle, le diméthylaminopropyl méthacrylamide et les sels de ceux-ci.

**10.** Polymère selon l'une des revendications précédentes, dans lequel la séquence comprenant les monomères de formule (I) comprend à la fois du méthacrylate d'isobornyle et de l'acrylate d'isobornyle.

**11.** Polymère selon l'une des revendications précédentes, dans lequel les monomères siliconés, seuls ou en mélange, sont présents à raison de 1 à 50% en poids, notamment à raison de 2 à 40% en poids, voire de 2,5 à 35% en poids, par rapport au poids total du polymère.

**12.** Polymère selon l'une des revendications précédentes, dans lequel les monomères de formule (I), seuls ou en mélange, sont présents en une quantité de 20 à 80% en poids, notamment 25 à 70% en poids, encore mieux 30 à 60% en poids, par rapport au poids total du polymère.

**13.** Polymère selon l'une des revendications précédentes, comprenant une séquence de Tg supérieure ou égale à 20°C, par exemple comprise entre 20 °C et 160 °C.

**14.** Polymère selon l'une des revendications précédentes, présentant un indice de polydispersité supérieur à 2, par exemple allant de 2 à 9, de préférence supérieur ou égal à 2,5, par exemple allant de 2,5 à 8, et mieux allant de 2,8 à 7.

**15.** Composition cosmétique ou pharmaceutique comprenant, dans un milieu physiologiquement acceptable, au moins un polymère tel que défini à l'une des revendications 1 à 14.

**16.** Composition selon la revendication 15, dans laquelle le polymère est présent en une quantité de 0,01 à 50% en poids, de préférence 0,05 à 40% en poids, notamment 0,1 à 30% en poids, voire 0,5 à 20% en poids, et encore mieux 1 à 15% en poids, préférentiellement 1,5 à 12% en poids, par rapport au poids total de la composition.

**17.** Composition selon l'une des revendications 15 à 16, dans laquelle le polymère est présent sous forme solubilisée ou sous forme de dispersion, par exemple dans une huile et/ou un solvant organique carboné.

**18.** Composition selon l'une des revendications 15 à 17, dans laquelle le milieu physiologiquement acceptable comprend au moins un constituant choisi parmi l'eau, les solvants organiques hydrophiles, les cires, les corps gras pâteux, les gommes et leurs mélanges; les solvants organiques lipophiles; les huiles, notamment hydrocarbonées d'origine animale ou végétale, ou siliconées; les pigments, les nacres, les charges, les colorants hydrosolubles, les colorants liposolubles; les polymères notamment filmogènes; les vitamines, les épaississants, les gélifiants, les oligo-éléments, les adoucissants, les séquestrants, les parfums, les agents alcalinisants ou acidifiants, les conservateurs, les filtres solaires, les tensioactifs, les anti-oxydants, les agents anti-chutes des cheveux, les agents anti-pelliculaires, les agents propulseurs, les céramides, les agents auxiliaires de filmification, ou leurs mélanges.

**19.** Composition selon l'une des revendications 15 à 18, se présentant sous la forme d'un produit de soin et/ou de maquillage de la peau du corps ou du visage, des lèvres, des ongles, des cils, des sourcils et/ou des cheveux, d'un produit solaire ou autobronzant, d'un produit capillaire pour le soin, le traitement, la mise en forme, le maquillage ou la coloration des cheveux.

**20.** Composition selon l'une des revendications 15 à 19, se présentant sous la forme d'une composition de maquillage, notamment un rouge à lèvres.

**21.** Procédé de traitement cosmétique, notamment de maquillage ou de soin des matières kératiniques telles que la peau du corps ou du visage, des lèvres, des ongles, des cils, des sourcils et/ou des cheveux, comprenant l'application sur lesdites matières d'une composition cosmétique telle que définie à l'une des revendications 15 à 20.

Office européen des brevets

# RAPPORT DE RECHERCHE EUROPEENNE

Numéro de la demande
EP 07 11 8609

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | EP 1 690 526 A (OREAL [FR]) 16 août 2006 (2006-08-16) * revendications; exemples * ----- | 1-21 | INV. C08F265/04 C08F293/00 A61K8/72 A61K8/90 |
| A | US 5 314 961 A (ANTON WAIFONG L [US] ET AL) 24 mai 1994 (1994-05-24) * revendications; exemples * ----- | 1-21 | |
| A | US 5 371 147 A (SPINELLI HARRY J [US] ET AL) 6 décembre 1994 (1994-12-06) * revendications; exemples * ----- | 1-21 | |
| A | BELLAS V ET AL: "Evaluation of siloxane and polyhedral silsesquioxane copolymers for 157 nm lithography" JOURNAL OF VACUUM SCIENCE AND TECHNOLOGY. B, MICROELECTRONICS AND NANOMETER STRUCTURES PROCESSING, MEASUREMENT AND PHENOMENA, AMERICAN INSTITUTE OF PHYSICS, NEW YORK, NY, US, vol. 20, no. 6, novembre 2002 (2002-11), pages 2902-2908, XP002438753 ISSN: 1071-1023 * figures 2,4a * ----- | 1-21 | DOMAINES TECHNIQUES RECHERCHES (IPC) C08F |

Le présent rapport a été établi pour toutes les revendications

| Lieu de la recherche | Date d'achèvement de la recherche | Examinateur |
|---|---|---|
| Munich | 19 février 2008 | IRAEGUI RETOLAZA, E |

3

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 07 11 8609

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

19-02-2008

| Document brevet cité au rapport de recherche | Date de publication | Membre(s) de la famille de brevet(s) | Date de publication |
|---|---|---|---|
| EP 1690526 A | 16-08-2006 | CN 1853611 A | 01-11-2006 |
| | | FR 2881950 A1 | 18-08-2006 |
| | | JP 2006225389 A | 31-08-2006 |
| US 5314961 A | 24-05-1994 | AUCUN | |
| US 5371147 A | 06-12-1994 | AUCUN | |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 1411069 A


### Littérature non-brevet citée dans la description


- Polymer Handbook. John Wiley, 1999